# EUROPEAN PATENT SPECIFICATION

(11) **EP 2 126 084 B1**
(45) Date of publication and mention of the grant of the patent: **26.03.2014**
(21) Application number: 07858225.1
(22) Date of filing: 28.12.2007
(51) Int. Cl.: C12N 15/11, C07K 14/47

(54) **POLYNUCLEOTIDE SEQUENCE FOR THE INHIBITION OF PHOSPHOLAMBAN SYNTHESIS**
POLYNUKLEOTID-SEQUENZ ZUR HEMMUNG DER PHOSPHOLAMBAN-SYNTHESE
SÉQUENCE POLYNUCLÉOTIDIQUE POUR L'INHIBITION DE LA SYNTHÈSE DU PHOSPHOLAMBAN

(30) Priority: 29.12.2006 EP 06090225
(43) Date of publication of application: 02.12.2009
(73) Proprietor: Charité - Universitätsmedizin Berlin, 10117 Berlin (DE); Freie Universität Berlin, 14195 Berlin (DE)
(72) Inventor: FECHNER, Henry, 15926 Luckau (DE); KURRECK, Jens, 12205 Berlin (DE); PRÖMEL, Simone, 12159 Berlin (DE)
(74) Representative: Gross, Felix
(86) International application number: PCT/EP2007/064637
(87) International publication number: WO 2008/080985

(56) References cited:
- EP-A1- 0 309 237
- WO-A-2006/096441
- FECHNER H ET AL P ET AL: "Highly efficient and specific modulation of cardiac calcium homeostasis by adenovector-derived short hairpin RNA targeting phospholamban" GENE THERAPY, vol. 14, no. 3, 5 October 2006 (2006-10-05), pages 211-218, XP002430901 ISSN: 0969-7128
- "Block-it TM Pol II miR RNAi Expression Vektor Kits" [Online] 20 November 2006 (2006-11-20), INVITROGEN , XP002430905 Retrieved from the Internet: URL:www.invitrogen.com> [retrieved on 2007-04-24] the whole document
- MULLER ET AL: "Improved cardiac gene transfer by transcriptional and transductional targeting of adeno-associated viral vectors" CARDIOVASCULAR RESEARCH, XX, XX, vol. 70, no. 1, 1 April 2006 (2006-04-01), pages 70-78, XP005350270 ISSN: 0008-6363 cited in the application
- DATABASE EMBL [Online] 15 December 2004 (2004-12-15), "Mus musculus BAC clone RP23-454L5 from 1, complete sequence." XP002472303 retrieved from EBI accession no. EMBL:AC153653 Database accession no. AC153653
- DATABASE Geneseq [Online] 5 April 2007 (2007-04-05), "p52 siRNA Neg." XP002472304 retrieved from EBI accession no. GSN:AER58403 Database accession no. AER58403
- DATABASE EMBL [Online] 29 June 2006 (2006-06-29), "Aotus nancymaae clone CH258-338D12, WORKING DRAFT SEQUENCE, 8 ordered pieces." XP002472305 retrieved from EBI accession no. EMBL:AC187935 Database accession no. AC187935

## Description

This invention relates to reagents that facilitate the inhibition of phospholamban in heart tissue of higher animals. More specifically, the invention relates to polynucleotide sequences capable of down-regulating phospholamban expression in a patient's cell, as specified in the independent claims.

Cardiac insufficiency and congestive heart failure are pathologies of major importance. They are associated with deficiencies in cardiac contractility and abnormalities in intracellular calcium handling.

Phospholamban (PLB) is a 52 amino acid integral membrane protein that takes part in the regulation of the calcium ion pump in muscle cells. In heart muscle cells, PLB controls the sarco-endoplasmatic reticulum Ca²⁺ ATPase pump (SERCA). The PLB/SERCA ratio influences cardiac contractility. Increased expression of SERCA and inhibition of PLB both work towards improved contractility in failing cardiomyocytes. The mRNA (cds) of phospholamban is published in the National Center of Biotechnology Information (NCBI) nucleotide database as NM_002667.2.

It has been shown that over-expressing SERCA can restore contractility, calcium handling and frequency response in isolated cardiomyocytes (Del Monte et al., Circulation 1999, 100:2308-2311).

Different approaches are possible for down-regulating the PLB activity in a cell. US2004121942A1, for example, employs expression of exogenous mutated PLB protein to attain this effect.

Another approach is the use of ribonucleic acid (RNA) such as antisense or siRNA to suppress gene expression at the level of mRNA control.

This is for instance described in WO 2008/080985, which discloses several siRNA molecules corresponding to a portion of a phospholamban nucleic acid sequence, which are able to inhibit the expression of phospholamban in a cell. This document also mentions that it could be possible to use an expression vector comprising DNA encoding said siRNA, whereby the vector can be an adenoviral vector.

Since RNA is not a stable molecule that lends itself easily to pharmaceutical formulation and use, direct application of RNA molecules is not necessarily the most promising clinical approach to apply RNA to the management of human disease. One approach circumventing RNA instability is the use of chemically modified ribonucleotides, such as thiophosphate backbone molecules or 2'deoxy purine or 2'-O-methly pyrimidine moieties. Comparable approaches during the antisense era have suffered from toxicity issues caused by the molecules and their degradation products.

An alternative approach that avoids application of RNA molecules is to transcribe RNA within the patient's cell from deoxyribonucleic acid (DNA) expression cassettes. DNA is more stable under many conditions encountered in drug delivery, and different technologies are known to deliver DNA expression cassettes into the cells of patients. These include viral transfer, particle-mediated bombardment of cells, injection of naked DNA or DNA complexed by polycations or liposomes into tissue, and other methods known in the context of so-called "gene therapy".

It has been attempted to restore contractility of failing heart muscle cells *in-vitro* by inhibiting PLB expression with antisense RNA molecules expressed from adenoviral vectors in cultured cardiomyocytes (Eizema et al., Circulation 2000; 101; 2193-2199; and Del Monte et al., Circulation 2002; 105; 904-907).

Different enzymes transcribe RNA from DNA in a eukaryotic cell, specifically, RNA polymerase I, II and III. Transcription from each polymerase is controlled by different regulatory elements regulating the expression of the associated gene. Traditionally, expression of short, non-mRNA molecules from expression cassettes artificially introduced into cells have made use of RNA polymerase III (Pol-III). Pol-III synthesizes 5S ribosomal and tRNA. The activity of RNA Pol-III is not controlled in a cell-specific fashion. US2005064489 describes Pol-III promoters regulated by the tetracycline transactivator system. Such conditionally active promoters offer the advantage of being able to control the state of activity of the artificially introduced transgene in a patient; they do not, however, offer cell specificity if applied to the entire body of a patient by systemic delivery.

In any clinical approach that attempts to regulate gene expression, cell or tissue specificity is a highly desirable element of control and safety. Hence, means of facilitating PLB-targeted RNA expression from cell-specific promoters such as RNA polymerase II promoters for the control of phospholamban expression in heart cells would be advantageous.

Antisense regulation of PLB from Pol-II promoters has been attempted by expressing antisense RNA under the control of the CMV or the inducible atrial natriuretic factor (ANF) promoter (Eizema et al., Circulation 2000; 101; 2193-2199).

Attempts to down-regulate expression of intracellular proteins by antisense approaches have generally not achieved the clinical successes expected from the technology at the time of its inception. A new approach to down-regulate intracellular gene expression is the use of so-called small interfering RNA (siRNA) molecules. These are pairs of short, double-stranded RNA molecules typically 19-29 base pairs in length, one strand of which is complementary to a section of mRNA. The targeted mRNA is degraded by the RISC complex. siRNA molecules were originally discovered in plants (Hamilton and Baulcombe, Science. 1999 Oct 29;286(5441):886), and later mammalian cells (Elbashir et al., Nature. 2001 May 24;411 (6836):428-9, WO0244321).

Closely related is the discovery of microRNA or miRNA. These are RNA molecules naturally synthesized from genomically encoded genes that, upon processing by cellular RNA-modifying activities, render short RNA hairpin structures with 11 bp (basepair) stem structures. miRNA also targets mRNA for degradation and is probably a mechanism of gene expression control within the cell.

US26198825A1 shows siRNA sequences that are suggested to target phospholamban synthesis.

One approach to the generation of siRNA in-vivo is the generation of so-called small hairpin RNA (shRNA), which is a natural precursor of siRNA. Such shRNA molecules can be generated by expression of a partially self-complementary RNA molecule from an expression cassette introduced into a eukaryotic cell. The technology is reviewed by McIntyre and Fanning (BMC Biotechnol. 2006; 6:1).

We have recently published the results of an *in-vitro* experiment, whereby PLB-mRNA and protein is reduced, and Ca uptake is increased, in cultured primary neonatal rat cardiomyocytes by shRNA expressed from polymerase-III-promoter-driven adenoviral constructs (Fechner et al., Gene Therapy 2007, 14, 211-218).

Additional technical obstacles exist to express shRNA from Pol-II promoters. This problem recently led to the development of so-called misiRNA (microRNA-based siRNA) generating vectors that can be used to drive siRNA-generation from a Pol-II-promoter (Shin et al., Proc. Nat. Acad. USA 103, 13759-13764). misiRNA can be described as an shRNA sequence enbedded between particular, microRNA-flanking sequences. This technology has recently been made available by Invitrogen Inc. as the pcDNA6.2-GW/miR plasmid. This plasmid comprises the CMV promoter (cytomegalocirus immediate early promoter) as the Pol-II expression element. The CMV promoter drives polymerase II transcription in a constitutive, tissue-non-specific fashion. The generation of misiRNA sequences from shRNA sequences is not

Different viral vectors can be employed for gene transfer into a patient's cells. Retroviruses, especially lentiviruses have been employed. Lentivirus-mediated gene transfer can lead to stable integration of a transgene into a non-dividing cell. Herpesvirus family members have also been employed for gene transfer.

As discussed above, for the purpose of systemic delivery of an agent that induces transgene expression in a patient, tissue specificity of expression is highly desirable. Cell type- or tissue-specific Pol-II promoters are known for different tissues. A disadvantage of the vast majority of these promoter sequences is, however, their length. DNA sequences over a certain size are difficult or impossible to use for gene therapy approaches making use of plasmid or viral transgene vectors, as the latter tend to tolerate only a limited size range for transgene sequences.

Moreover, in vivo delivery of shRNAs from a constitutive polymerase III promoter from an AAV vector can lead to death of exprimental animals through acute liver toxicity. An evaluation of 49 distinct AAV/shRNA vectors, unique in length and sequence and directed against six targets, showed that 36 resulted in dose-dependent liver injury, with 23 ultimately causing death. (Grimm et al., Nature, 441, 537-541)

Thus, an expression system for the expression of an RNA molecule that can lead to the down-regulation of phosholamban expression in a cell of the heart, in a cell-type specific manner, would be desirable.

One objective of the invention is to provide a reagent that can be applied to a patient suffering from a condition associated with the activity of phospholamban, or a condition which could benefit from reduction of phospholamban expression, where the reagent, upon application to a patient, leads to the down-regulation of phospholamban synthesis in heart cells of the patient in a cell-type specific manner.

Another objective is to provide a novel RNA molecule that can lead to the down-regulation of phosholamban expression in a cell of the heart.

These objectives are attained by the invention as it is specified in the independent claims.

According to one aspect of the invention, a DNA polynucleotide sequence is provided comprising a.
a. a first sequence element that when transcribed by an RNA polymerase leads to the transcription of a microRNA-based siRNA (misiRNA) and renders a transcript capable of forming a partially self-complementary hairpin structure which can be processed by a mammalian cell to an siRNA product capable of degrading an mRNA in said cell, whereby the mRNA degraded by the siRNA product encodes phospholamban,
b. a promoter sequence operably linked to the first sequence element, said promoter sequence being operable by an RNA polymerase naturally occurring in a mammalian cell, whereby the promoter sequence is a cardiomyocyte cell-specific promoter, and
c. a virus-derived DNA polynucleotide packaging sequence.

Wherein the misiRNA comprises at least one of the sequences SEQ ID NO 001, SEQ ID NO 002 or SEQ ID NO 003.

"Self-complementary" in the context of this document means that the RNA molecule has two sequence tracts that allow canonical base-pairing in 5' to 3' direction of one sequence tract with the other in reverse orientation, so that the RNA linear polymer can fold back on itself to produce a RNA double-helical structure. Such hairpin RNA molecule, formed for example by transcribing a transgene DNA sequence with a DNA-dependent RNA polymerase in a cell, may be processed by intracellular RNA-processing activities to yield a functional siRNA molecule capable of down-regulating the expression of the mRNA it is targeted to, which in case of the present invention is phospholamban mRNA.

Surprisingly it was found that the misi RNA sequences of the present invention targeted to phosholamban, were around two orders of magnitude more efficient in suppressing PLB expression than the sh RNAs of the state of the art as shown in Fechner et al. (Gene Therapy 2007, 14, 211-218).

The hairpin structure of the misi RNA molecules of the instant invention can also be defined as one of its hybridizing strands being highly similar to a sequence comprised in the phospholamban mRNA sequence, the GeneBank reference for which is given above. Sequence similarity quantification is well known in the art of molecular biology; computational tools for quantification of sequence similarity are found, among other places, at the EMBL-EBI website at http://www.ebi.ac.uk/Tools/similarity.html. According to the invention, the hybridizing sequences of the RNA molecule can be 80%, 85%, 90%, 95%, 97% or 100% similar to a sequence tract in phospholamban mRNA, for example the coding sequence of phospholamban mRNA; the 5' untranslated region or the 3' untranslated region.

According to another aspect of the invention, an expression cassette for the intracellular expression of a phospholamban-specific inhibitory RNA is provided, which can be processed by the cell in which it is expressed, to down-regulate the expression of phospholamban synthesis by that cell. Such expression cassette comprises a promoter sequence operable in a mammalian cell by a mammalian RNA polymerase, and a transcribed sequence, also referred to as a first sequence element.

An expression cassette is preferred where the promoter sequence operable in a mammalian cell is a cardiac tissue specific DNA-dependent RNA polymerase II promoter. Examples of such preferred promoters are the sequences SEQ ID 7, 8 and 9. For use of the expression cassette in transgene transfer systems of limited capacity such as plasmid vectors and AAV vectors, short sequences are preferred. The promoter element MLC 260, which comprises the CMV enhancer and a cardiac tissue specific core sequence of the MLC promoter and is only 850 (ca. 600 CMV enhancer, ca. 260 MLC core promoter) base pairs long, is especially preferred.

The transcribed sequence or first sequence element comprises a DNA sequence, which, when transcribed by an-RNA polymerase in a cell, renders a transcript capable of forming a partially self-complementary hairpin structure that can be processed by the cell to yield an siRNA product. Such siRNA product typically is a 19 to 29 base pair long RNA double strand with short overhangs on the 3' end, capable of degrading an mRNA in said cell. According to the invention, the hairpin structure and the resulting siRNA product is at least partially complementary to a section of the phospholamban mRNA sequence synthesized in that cell.

Specifically, according to one aspect of the invention, a transcribed sequence or first sequence element leads to the transcription of a microRNA-based siRNA (misiRNA) molecule that can be processed by the cell to yield a siRNA molecule targeting PLB-mRNA.

In the context of the instant invention, "a promoter being operable by an RNA polymerase" means that the presence of the promoter sequence characterized as "a promoter" is known to lead to recruitment of the necessary cellular factors and the recruitment and initiation of transcription of said RNA polymerase. "Operably linked" in the context of this specification means that a promoter sequence and a transcribed sequence are linked in such way that the promoter will, under physiological conditions, lead to the transcription of the transcribed sequence. Hence, a "Pol-II-promoter" under physiological conditions is able to recruit RNA polymerase II to the transcribed sequence operably linked thereto, and lead to the initiation of transcription of that transcribed sequence.

If the promoter is a Pol-II promoter, the transcribed sequence preferably comprises additional sequence elements that facilitate the processing of the initial Pol-II-derived transcript by the splicing apparatus in the cell, to yield a misi RNA molecule that is able to target PLB-mRNA and to down-regulate the expression of PLB in a cell in which the expression cassette is transcribed.

As mentioned misi RNA sequences that have been employed according to the invention are SEQ ID NO 001, SEQ ID NO 002 and SEQ ID NO 003 The bases no. 6 to 26 of these sequences comprise, when transcribed into RNA, the part of the small hairpin RNA that will later form the mature siRNA directed against the target mRNA. These sequences are underscored below, with their respective complementary part on the 3' end in boldface:

Sequences are known that serve as signals to direct the processing of RNA molecules by the splicing apparatus of the cell. Splicing is catalyzed by the spliceosome, which is a large RNA-protein complex composed of five small nuclear ribonucleoproteins present in eukaryotic cells. Examples for additional sequence elements that facilitate the processing of the initial Pol-II-derived transcript by the processing and splicing apparatus in the cell to yield a mature shRNA molecule are splice donor and splice acceptor sites. Examples for additional sequence elements that facilitate the processing of the initial Pol-II-derived transcript, and which have successfully been employed in the context of the present invention, are the 5' miR flanking region (SEQ ID NO 004) and the 3' miR flanking region (SEQ ID NO 005). Transcribed sequences that comprise a shRNA-generating element and splice donor and acceptor sites for liberation of that transcribed element are referred to as misiRNA (from micro-siRNA) in the context of this invention.
SEQ ID NO 004: ctggaggctt gctgaaggct gtatgctg
SEQ ID NO 005: aggacacaag gcctgttact agcactcaca tggaacaaat ggccc

A transcribed sequence comprising a transcription initiation sequence for Pol-II and the transcribed region (the + strand) including splice donor and acceptor sites and the shRNA element are shown in SEQ ID NO 006.

According to one aspect of the invention, transcription of the transcribed sequence can be effected by a RNA-polymerase II (Pol-II) promoter. One such promoter is the Cytomegalovirus immediate early promoter, commonly referred to as CMV promoter. Other commonly employed promoters are the simian virus 40 (SV40) promoter, Rous Sarcoma Virus (RSV) promoter and EF1alpha promoter. According to one aspect of the invention, a Pol-II promoter is preferred that is specific for cardiomyocytes. "Specific" in the context of the present invention means that an expression cassette comprising the promoter that is specific for cardiomyocytes and the first sequence element operably linked thereto, is transcribed in cardiomyocytes at a significantly higher rate than in other cell types. An example for a cardiomyocyte-specific promoter are the sequence MLC1500 (SEQ ID No 007) MLC800 (SEQ ID No 008) (Muller et al. 2006, Cardiovascular research, 70, 70-78.) and MLC260 (SEQ ID No 009).

According to another aspect of the invention, a conditional Pol-II promoter is preferred that can be induced in the patient, preferably by administration of a small molecule pharmaceutical compound. One example for such conditional promoter is the tetracycline transactivator system reviewed by Gossen and Bujard (Ann. Rev. Genet. 2002, 36:153-73). In this "tet-on" system, gene expression is regulated by presence of tetracycline or doxycycline by binding of the tetracycline transactivator protein to tetracycline response elements on the DNA. A conditional promoter in the context of the present specification shall signify a promoter the activity of which can be significantly increased by modifying directly or indirectly a parameter extrinsic to the cell in which the promoter is active. Non-limiting examples for such conditional promoters are promoters with different activities depending on the concentration of a small molecule drug, or depending on the temperature of the cell, or the ionic strength of a specific ion in the cell or its surrounding.

A sequence comprising the conditional promoter of the tet-on-system and a transcribed sequence yielding a misiPLB-shRNA molecule and comprising the TRE-tight1 promoter construct (Sipo et al. 2006, JMM, 84:215-225) is shown in SEQ ID NO 010. Therein, bases no. 1-252 comprise the TetO7 site, bases 253 -314 are the CMV minimal promoter sequence with base 306 (thymine) being the putative transcriptional start site, and from base 394 to 453 the misiPLBh-shRNA flanked by the 3' miR flanking region, the 5' miR flanking region and the SV40 polyadenylation sequence.

Similarly, a TRE-tight2 promoter as described by Sipo et al. (as cited above) can be employed.

According to another aspect of the invention, the expression cassette is administered to a patient in form of a viral vector. According to one aspect of the invention, an adenovirus is used to mediate the transfer of the expression cassette for the expression of an RNA molecule for PLB down-regulation in a cell. An adenovirus type 5 is preferred. Certain adenovirus subtypes are known to exhibit tropism for heart muscle cells and can thus be employed to transfer the inventive expression cassette into cardiomyocytes. Adenovirus type 5 is one example for a virus exhibiting heart tissue tropism.

Methods and experimental protocols for producing adenovirus virions comprising an expression cassette for therapeutic or vaccination purposes are known in the art (for a current review, see Bangari and Mittal, Curr Gene Ther. 2006 Apr;6(2):215-26). Adenovirus vectors can be produced by transfection of an adenoviral DNA into a helper cell line (e.g HEK 293) which expresses adenoviral proteins (e.g. E1A, E1B), that can not be expressed from the adenoviral genome as the respective genes were deleted from the genome. An adenoviral vector in the context of the present specification thus means a DNA sequence comprising an expression cassette as specified above, comprising the following elements:
- a promoter operable in a mammalian myocardial cell,
- a transcribed first sequence element comprising a misi RNA-generating sequence that upon transcription will render a RNA transcript, which can be processed by the cellular RNA processing apparatus into an siRNA directed against PLB-mRNA, and
- the virus-derived sequences necessary to effect packaging of the DNA sequence into virion particles by a suitable packaging cell line for production of replication-defective adenovirus virions containing the expression cassette.

According to another aspect of the invention, an adeno-associated virus (AAV) is used to mediate the transfer of the expression cassette for the expression of an RNA molecule for down-regulation of PLB in a cell. AAV are non-pathogenic and lead to little if any immune reaction against them. Expression of AAV-borne expression transgenes is stable over a long time period.

Certain AAV subtypes are known to exhibit tropism for heart muscle cells and can thus be employed to transfer the inventive expression cassette into cardiomyocytes. AAV9 is one example for a virus exhibiting heart tissue tropism.

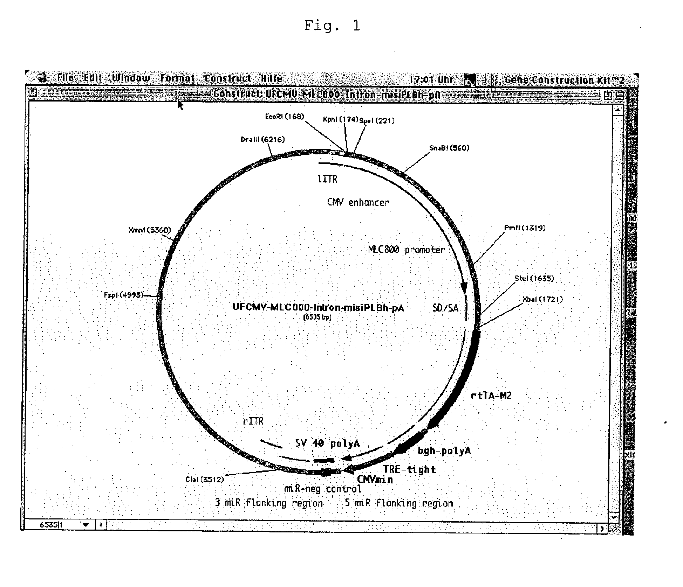

AAV9 vectors are produced by co-transfection methods. The vector genome comprises the ITR (inverted terminal repeat) sequences of AAV2 or ITR sequences of other AAVs adjacent to the expression cassette for the expression of the transgenes (Plasmid: UFCMV-MLC800-Intron-misiPLBh-pA). The AAV genome is packaged into an AAV9 capsid (Plasmid: p5E18-VD2/9; it contains the AAV-Rep2 gene + CAP9 gene from AAV, a kind gift from Dr. Wilson, University of Pennsylvania, USA). Further proteins essential for packaging are derived from Plasmid 3 (e.g. E1A, VA-RNA of adenovirus). AAV vector particles are produced in cultured cells and released from cell culture by three freeze/thaw cycles and caesium gradient centrifugation (Grimm: Methods (2002), 28, 146-157). One example of an AAV vector genome containing tetracycline regulated misiPLBshRNA expression cassette is shown in SEQ ID NO 011 (for the vector chart, see Fig. 1).

AAV can be produced as single stranded or self complementary (sc) AAV vectors. A scAAV vector contains a small mutation in one terminal resolution site, making it possible to package a dimeric vector genome. One example for a scAAV vector comprising an inventive misi-RNA construct able to down-regulate phospholamban mRNA is shown in SEQ ID NO 012 (scAAV-CMV800 misiPLBh)

According to one preferred embodiment, an expression cassette comprising a misiRNA for the generation of an siRNA molecule targeting PLB expression, under the control of a tet-on conditional promoter system and comprising the packaging sequence elements for packaging the expression cassette into an AAV vector is shown in SEQ ID NO 011.

According to yet another aspect of the present invention, an adeno-associated-virus virion comprising the expression cassette of the previous paragraph is provided. Preferably, this virion is an AAV type 9 (AAV9) virion. Similarly, a composition for the treatment of cardiomyopathy comprising an expression cassette as disclosed in the previous paragraph, or a virion as disclosed in this paragraph, is provided. Such virion may be obtained by co-transfecting a suitable expression cassette such as the expression cassette of the previous paragraph or specifically, an expression cassette as exemplified by SEQ ID NO 011, into a suitable cell line, along with a plasmid containing the elements for packaging the expression cassette into AAV9 virions.

Methods and experimental protocols for producing adeno-associated virus virions comprising an expression cassette for therapeutic or vaccination purposes are known in the art. (Grimm, Methods, 28, 146-157). An AAV vector in the context of the present specification thus means a DNA sequence comprising an expression cassette as specified above, comprising a promoter operable in a mammalian myocardial cell, and a transcribed sequence comprising a misi RNA-generating RNA transcript able to render an siRNA directed against PLB-mRNA, and the adeno-associated virus-derived sequences necessary to effect packaging of the DNA sequence into a suitable packaging cell line for production of AAV viral particles containing the expression cassette.

According to yet another aspect of the present invention, an improved conditional promoter is provided for the use in an expression cassette controlling the transcription of short hairpin RNA that can be processed into siRNA in the cell. The tet-on system currently in use consists of a CMV promoter driving the expression of a recombinant tetracycline transactivator protein (rtTA), which, in turn, binds to down-stream tetracycline response elements (TRE) on the same DNA strand, leading to expression of a transcribed sequence under control of the TRE. In theory, transcription of the transcribed sequence only proceeds when tetracycline or doxycycline is present. The system, however, suffers, in our experience, from constitutive expression in the absence of the inducing drug, probably because the very strong CMV promoter "reads through" the entire downstream sequence and produces a transcript that includes the downstream misiRNA-generating sequence, thus leading to misiRNA production from the CMV promoter instead of the drug-controlled TRE elements. On the other hand the leakiness of the TRE element may be inhibited by replacement of the minimal CMV (CMVmin) promoter through another non leaky promoter such as as tight2 (see above for sequence details and sequence data contained herein), but also by use of an tetracycline-controlled transcriptional silencer (tTS) which binds to the TetO7 and represses the CMVmin (Fechner et al. 2003, Gene Therapy, 2003, 10, 1680-1690).

According to the invention, this problem can be circumvented by exchanging the CMV promoter driving the expression of the rtTA protein, for a cell-specific, less active promoter such as MLC260 (SEQ ID NO 009)

According to another preferred embodiment, an expression cassette comprising a transcribed element for the generation of a misiRNA molecule targeting PLB expression, under the control of a cardiomyocyte-specific promoter in an AAV packaging vector is provided.

An AAV vector in the context of the present specification is the preferred embodiment of the invention. It comprises a DNA sequence a polynucleotide sequence as specified above, comprising the following elements:
- a promoter operable in a mammalian myocardial cell, preferably a myocard-specific promoter, more preferably a myocard-specific conditional promoter such as the tet-on system under control of the MLC260 promoter,
- a transcribed first sequence element comprising a misiRNA-generating sequence that upon transcription will render a RNA transcript, which can be processed by the cellular RNA processing apparatus into an siRNA directed against PLB-mRNA, and
- the virus-derived sequences necessary to effect packaging of the DNA sequence into virion particles by a suitable packaging cell line for production of replication-defective AAV virions, preferably AAV9 virions, containing the expression cassette.

Alternatively, according to yet another aspect of the invention, the expression cassette for the expression of an RNA molecule for down-regulation of PLB in a cell can be applied to the patient as a naked DNA or as DNA in association with a non-viral transfer agent, for example encapsulated in liposomes or in association with polyamine reagents such as polyethylenimine. Expression cassettes either in association with a virus, with a nonviral transfer agent or as naked DNA can be applied by intravenous injection, intramuscular injection, particle-mediated gene transfer or any other suitable transfer method.

According to yet another aspect of the present invention, an expression construct expressing an inhibitory RNA construct such as described above, can be combined with an expression construct facilitating an increased expression of SERCA. Methods for expressing polypeptides in human cells are well known in the art, and include the methods of viral, liposomal and naked DNA transfer discussed above. mRNA can be expressed from SERCA coding sequences under control of Pol-II promoters such as CMV or inducible or cell-specific promoters, as discussed above. SERCA mRNA is published as GeneBank entry NM_170665.

According to the aforementioned aspect of the invention, an expression construct encoding SERCA may be located on the same polynucleotide sequence as the construct leading to phospholamban down-regulation. It may also be separated from the latter and packaged into the same or distinct virions, or associated into liposomal or cationic particles together with the phospholamban-specific shRNA generating construct.

Patients in the context of this invention can be humans, however it is apparent that the invention can also be applied to higher animals, such as dogs or horses as well as primates.

According to another aspect of the invention, the sequence of SEQ ID NO 009 can be used to drive transcription of any transgene, for example a therapeutic gene such as SERCA, an RNA construct or any other transcribed sequence, in a cardiac-cell-specific fashion.
- Fig. 1: shows a vector chart of a plasmid vector for packaging into AAV virions, containing an expression cassette comprising a cardiac cell-specific conditional (tet-on) promoter and a phosholamban mRNA targeting misiRNA construct. The sequence of the vector is given in SEQ ID NO 011.

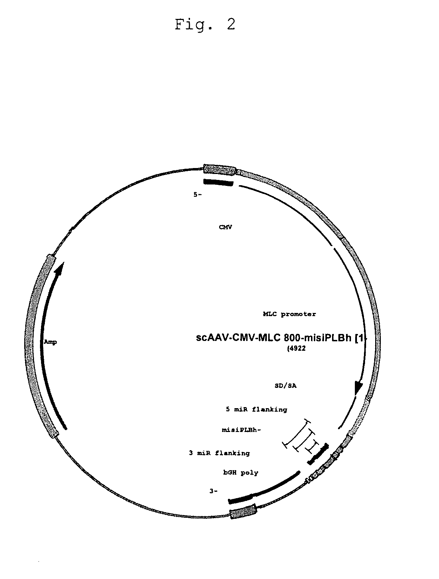

- Fig. 2: shows a vector chart of a plasmid vector for packaging into AAV virions, containing an expression cassette comprising a cardiac cell-specific promoter and a phosholamban mRNA targeting misiRNA construct. The sequence of the vector is given in SEQ ID NO 012.

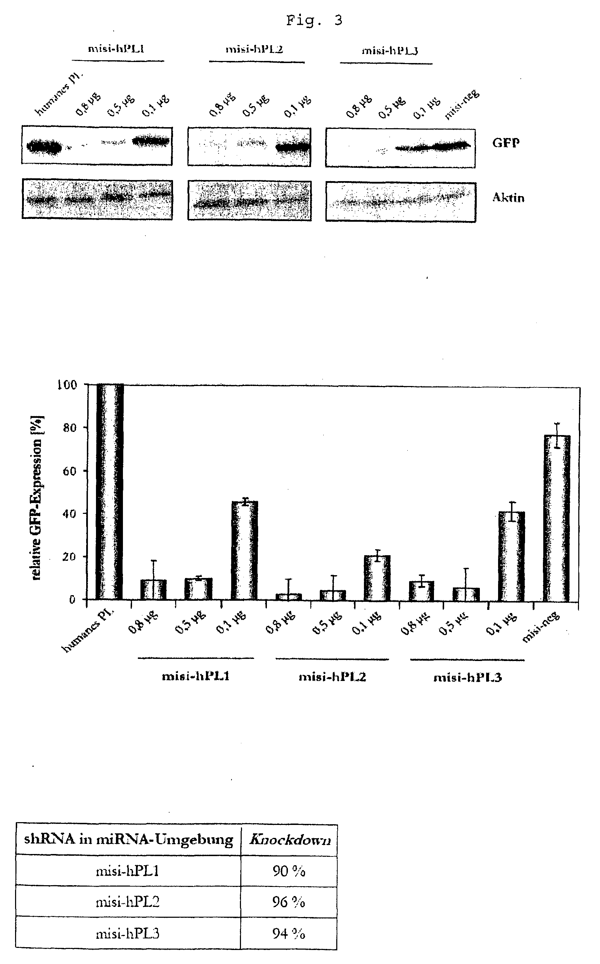

- Fig. 3: shows experimental results of shRNA expression against phospholamban mRNA in cells expressing a phospholamban-GFP-fusion construct. Specifically, Fig. 3 a and b show western blot results, Fig. 3c shows the expression inhibition efficacy as ratios of the densitometric evaluation of the western blots.

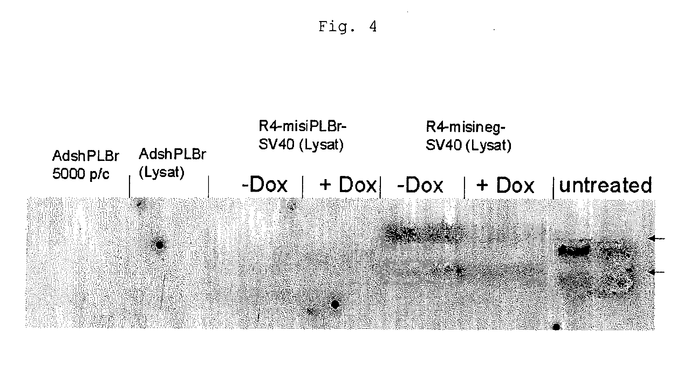

- Fig. 4: shows Northern blots with radioactive RNA transcripts blotted against PLB-mRNA from myocardiac cells (see Example 2). Lane 1-4: positive control, Adenovirus anti-PLB shRNA transcribed from an U6 promoter as published previously (Fechner et al. 2006 Gene Therapy Highly efficient and specific modulation of cardiac calcium homeostastis by adenovector-derived short hairpin RNA targeting PLB. DEU: 10.1038); lane 1,2: neonatal rat cardiomyocytes infected with caesium chloride gradient purified AdV particles expressing rat PLB-shRNA from an U6 (polymerase III promoter), lane 3, 4: neonatal rat cardiomyocytes infected with an HEK 293T cell lysate containing AdV particles expressing rat PLB-shRNA from an polymerase III promoter; lane 5-8 : neonatal rat cardiomyocytes infected with an HEK 293T cell lysate AdV particles expressing rat misiPLB-shRNA with and without doxycyclin, lane 9-12: negative control - neonatal rat cardiomyocytes infected with an HEK 293T cell lysate AdV particles expressing scambled-shRNA with and without doxycyclin, lane 13,14: neonatal rat cardiomyocytes (untreated) construct with and without doxocyclin, lane 9-12 : negative control (vector without misiRNA construct).

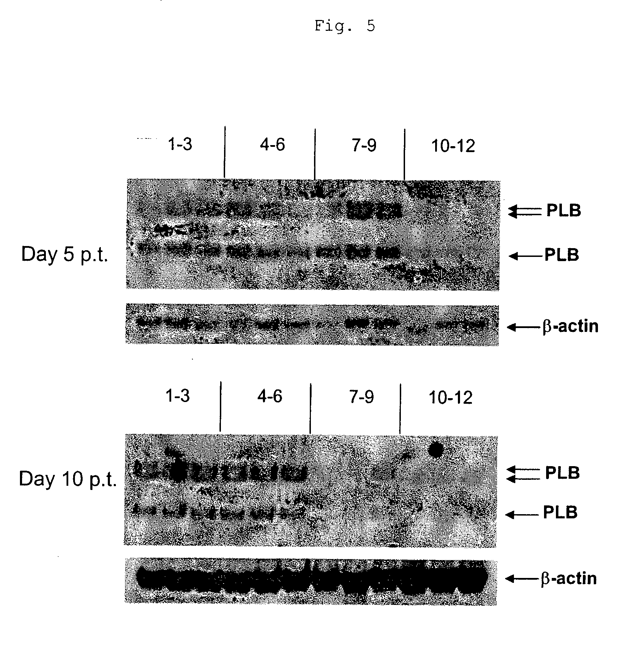

- Fig. 5: shows the results of a knockdown experiment on the expression of PLB-mRNA by scAAV (scAAV2.6-Vector) mediated rat-misiPLB delivery in neonatal rat cardiac myocytes. The upper panel shows results 5 days post vector transduction (p.t.), the lower panel results 10 days p.t. Three lanes each show Northern blots of untreated cells, cells treated with scAAV2.6-beta-shPLBr (lanes 4-6), cells treated with scAAV2.6-CMV-misiPLBr (lanes 7-9) and cells treated with scAAV2.6-MLC260-misiPLBr (Lanes 10-12). Lanes 1-3 untreated control cells.

### Examples

### Example 1: misiRNA expression down-regulates expression of human PLB in cos7 cells.

Cos7 cells were co-transfected with a plasmid expressing a GFP - human PLB fusion transcript (Fechner et al. 2006 Gene Therapy Highly efficient and specific modulation of cardiac calcium homeostastis by adenovector-derived short hairpin RNA targeting PLB. DEU: 10.1038). Cells were harvested 48 h later and analyzed by Western-blot analysis for GFP expression. All human misiPLB-shRNA exhibited down-regulated GFP expression in a dose-dependent manner, indicating that the expression of the fusion construct was silenced by the misiPLB-shRNA construct. See Fig 3.

### Example 2: Adenovirus mediated PLB knock-down by expression of misiPLB-shRNA

Neonatal rat cardiomyocytes were transduced with the respective adenoviral vectors or cell lysate from HEK293 containing adenoviral vector particles for 2h, medium was replaced and fresh medium added. Total RNA was isolated 4 days later and investigated for PLB-mRNA expression by Northern-blotting. Results (Northern-blot) are given in Fig. 4: Adenovirus vectors expressing rat PLB-shRNAs strongly suppressed PLB-mRNA expression. Similar silencing efficiency was found with the misiPLBr-shRNA expression vector R4-misiPLBr-SV40. However, PLB silencing was seen in the presence and absence of Doxycyclin (Dox), indicating probable leakiness or polymerase run-through from the upstream CMV promoter. Adenoviral vectors expressing a control misi-RNA (R4-misineg-SV40) was used as control.

A similar experiment is seen in Fig. 5: Self complementary (sc) AAV-vectors expressing PLB-shRNA (scAAV2.6-PLBr) were produced on 393 T cells by cotransfection of AAV2-derived scAAV shuttle plasmids and an AAV6 packaging plasmid. Newly generated vectors were released by three freeze-thaw cycles and cleaned by iodixanol gradient centifugation. The concentration of the vector was determined by southern-blotting.

In parallel, new misiPLBr expressing scAAV2.6 vectors were constructed. These vectors contain the heart specific MLC260 or an CMV promoter for transcription of misiPLB (scAAV2.6 CMV-misiPLBr and scAAV2.6 MLC-misiPLBr, resp.). Different to scAAB2.6-shPLBr vectors were released by three freeze/thaw cycles. After sedimentation of crude cell lysate, the vector-containing supernatant was directly used for transduction of the neonatal rat cardiac myocytes.

Neonatal rat cardiac myocytes were isolated and cultered for 48 h. Cells were infected with 5000 vector genomes per cells (scAAV2.6-shPLBr) and 500 µl/well (1.2 mill cells) of scAAV2.6 CMV-misiPLBr and scAAV2.6 MLC-misiPLBr, resp. For 24 h. RNA was isolated 5 and 10 days later and PLB mRNA expression determined by Northern-blotting

Results: AAV vectors expressing misiPLB were able to knock down PLB mRNA expression (Northern-blot hybridization, Fig. 5) 5 and 10 days after transduction. The efficiency of both CMV and MLC promoter driven misiPLBr was higher than for U6 promoter driven shPLBr in scAAV2.6-shPLBr (which was able to silence PLB expression in published experiments, see Fechner et al., ibid.).

Conclusion: misiPLBr can be expressed from polymerase II promoters (CMV and MLC) in a tissue specific manner (MLC) and is more efficient than PLB-shRNA in knocking down of PLB.

## Claims

1. DNA polynucleotide sequence comprising
a. a first sequence element that when transcribed by an RNA polymerase leads to the transcription of a microRNA-based siRNA (misiRNA) and renders a transcript capable of forming a partially self-complementary hairpin structure which can be processed by a mammalian cell to an siRNA product capable of degrading an mRNA in said cell, whereby the mRNA degraded by the siRNA product encodes phospholamban,
b. a promoter sequence operably linked to the first sequence element, said promoter sequence being operable by an RNA polymerase naturally occurring in a mammalian cell, whereby the promoter sequence is a cardiomyocyte cell-specific promoter, and
c. a virus-derived DNA polynucleotide packaging sequence.
**Characterized in that**
the misiRNA comprises at least one of the sequences SEQ ID NO 001, SEQ ID NO 002 or SEQ ID NO 003.

2. DNA polynucleotide sequence according to claim 1, **characterized in that** the cardiomyocyte cell-specific promoter sequence is an RNA polymerase II promoter.

3. DNA polynucleotide sequence according to claim 1 or 2, **characterized in that** the cardiomyocyte cell-specific promoter is at least one of the sequences SEQ ID NO 007, SEQ ID NO 008 or SEQ ID NO 009.

4. DNA polynucleotide sequence according to any of the preceding claims, **characterized in that** the first sequence element further comprises sequence elements that facilitate the processing of the transcript by the splicing apparatus of the cell.

5. DNA polynucleotide sequence according to any of the preceding claims, comprising at least one of the sequences SEQ ID NO 004 and SEQ ID NO 005 and at least one of the sequences SEQ ID NO 007, SEQ ID NO 008 or SEQ ID NO 009.

6. A DNA polynucleotide sequence according to any of the preceding claims, **characterized in that** the virus-derived DNA polynucleotide sequence is an adenovirus packaging sequence.

7. A DNA polynucleotide sequence according to any of the preceding claims, **characterized in that** the virus-derived DNA polynucleotide sequence is derived from an adeno- associated virus.

8. A DNA polynucleotide sequence according to claim 7, **characterised in that** the virus-derived DNA polynucleotide sequence is derived from adeno- associated virus type 9.

9. The DNA polynucleotide sequence of SEQ ID No 010 or SEQ ID NO 011 or SEQ ID NO 012.

10. A virus particle containing a polynucleotide sequence according to any of the preceding claims.

11. An isolated eukaryotic cell containing a polynucleotide sequence according to any of the claims 1 to 9.

12. An isolated eukaryotic cell according to claim 11, whereby the cell is a mammalian cell.

13. A composition for use in the treatment of cardiomyopathy comprising:
a. a polynucleotide sequence, a virus particle and / or an isolated mammalian cell according to any of the claims 1 to 11 and
b. a pharmaceutically acceptable excipient.

14. A composition for use in the treatment of cardiomyopathy comprising:
c. a polynucleotide sequence or a virus particle or an isolated mammalian cell according to any of the claims 1 to 11 and
d. a DNA polynucleotide sequence or a virus particle comprising an expression cassette comprising a second promoter sequence operable in a cardiomyocyte and a second sequence element encoding SERCA (sarco/endoplasmic reticulum Ca²⁺ -ATPase) operably linked to said second promoter sequence,
and
e. a pharmaceutically acceptable excipient.

## Patentansprüche

1. DNS-Polynukleotidsequenz umfassend
a. ein erstes Sequenzelement, das bei Transkription durch eine RNA-Polymerase zur Transkription einer mikroRNA-basierten siRNA (misiRNA) führt und in ein Transkript übersetzt, das fähig ist, eine teilweise selbstkomplimentäre Haarnadelstruktur auszubilden, welche durch eine Säugetierzelle in ein siRNA-Produkt prozessiert werden kann, das in der Lage ist, mRNA in der Zelle abzubauen, wobei die durch das siRNA-Produkt abgebaute mRNA für Phospholamban kodiert,
b. eine Promotorsequenz, die operativ mit dem ersten Sequenzelement verbunden ist, wobei die Promotorsequenz durch eine RNA-Polymerase, die natürlicherweise in einer Säugetierzelle vorkommt, gesteuert wird, wobei die Promotorsequenz ein Kardiomyozyt-zellspezifischer Promotor ist, und
c. eine Virus-abgeleitete DNA Polynukleotidverpackungssequenz,
**dadurch gekennzeichnet,**
**dass** die misiRNA mindestens eine der Sequenzen SEQ ID NO 001, SEQ ID NO 002 oder SEQ ID NO 003 umfasst.

2. DNS-Polynukleotidsequenz gemäß Anspruch 1, **dadurch gekennzeichnet, dass** die Kardiomyozyt-zellspezifische Promotorsequenz ein RNA Polymerase II Promotor ist.

3. DNS-Polynukleotidsequenz gemäß Anspruch 1 oder 2, **dadurch gekennzeichnet, dass** der Kärdiomyozyt-zellspezifische Promotor mindestens eine der Sequenzen SEQ ID NO 007, SEQ ID NO 008 oder SEQ ID NO 009 ist.

4. DNS-Polynukleotidsequenz gemäß einem der vorhergehenden Ansprüche, **dadurch gekennzeichnet, dass** das erste Sequenzelement des Weiteren Sequenzelemente umfasst, die die Prozessierung des Transkripts durch den Splicing-Apparat der Zelle ermöglichen.

5. DNS-Polynukleotidsequenz gemäß einem der vorhergehenden Ansprüche, umfassend mindestens eine der Sequenzen SEQ ID NO 004 und SEQ ID NO 005 und mindestens eine der Sequenzen SEQ ID NO 007, SEQ ID NO 008 oder SEQ ID NO 009.

6. DNS-Polynukleotidsequenz gemäß einem der vorhergehenden Ansprüche, **dadurch gekennzeichnet, dass** die Virus-abgeleitete DNS-Polynukleotidsequenz eine Andenovirus-Verpackungssequenz ist.

7. DNS-Polynukleotidsequenz gemäß einem der vorhergehenden Ansprüche, **dadurch gekennzeichnet, dass** die Virus-abgeleitete DNA Polynukleotidsequenz von einem adeno-assoziierten Virus abgeleitet ist.

8. DNS-Polynukleotidsequenz gemäß Anspruch 7, **dadurch gekennzeichnet, dass** die Virus-abgeleitete DNS-Polynukleotidsequenz von dem Adeno-assoziierten Virustyp 9 abgeleitet ist.

9. DNS-Polynukleotidsequenz gemäß SEQ ID NO 010 oder SEQ ID NO 011 oder SEQ ID NO 012.

10. Ein Viruspartikel enthaltend eine Polynukleotidsequenz gemäß einem der vorhergehenden Ansprüche.

11. Eine isolierte eukaryotische Zelle enthaltend eine Polynukleotidsequenz gemäß einem der Ansprüche 1 bis 9.

12. Isolierte eukaryotische Zelle gemäß Anspruch 11, wobei die Zelle eine Säugetierzelle ist.

13. Eine Zusammensetzung zur Verwendung in der Behandlung von Kardiomyopathie umfassend:
a. eine Polynukleotidsequenz, ein Viruspartikel und/oder eine isolierte Säugetierzelle gemäß einem der Ansprüche 1 bis 11 und
b. ein pharmazeutisch akzeptabler Hilfsstoff.

14. Eine Zusammensetzung zur Anwendung in der Behandlung von Kardiomyopathie umfassend:
c. eine Polynukleotidsequenz oder ein Viruspartikel oder eine isolierte Säugetierzelle gemäß einem der Ansprüche 1 bis 11 und
d. eine DNS-Polynukleotidsequenz oder ein Viruspartikel umfassend eine Expressionskassette umfassend eine zweite Promotorsequenz, die in einem Kardiomyozyten funktionsfähig ist, und ein zweites Sequenzelement für SERCA (sarco/endoplasmatisches Retikulum CA²⁺ -ATPase) kodiert und funktionsfähig mit der zweiten Promotorsequenz verbunden ist, und
e. ein pharmazeutisch akzeptierbarer Hilfsstoff.

## Revendications

1. Séquence polynucléotidique d'ADN comprenant
a. un premier élément de séquence qui lorsqu'il est transcrit par une ARN polymérase conduit à la transcription d'un ARNsi à base de microARN (ARNmisi) et rend un transcrit capable de former une structure en épingle à cheveux auto-complémentaire qui peut être traitée par une cellule de mammifère en un produit d'ARNsi capable de dégrader un ARNm dans ladite cellule, de telle manière que l'ARNm dégradé par le produit d'ARNsi code pour le phospholambane,
b. une séquence promoteur liée de manière opérationnelle au premier élément de séquence, ladite séquence promoteur pouvant être actionnée par une ARN polymérase existant à l'état naturel dans une cellule de mammifère, de telle manière que la séquence promoteur est un promoteur de spécificité cardiomyocytaire, et
c. une séquence d'encapsidation de polynucléotides d'ADN d'origine virale,
**caractérisée en ce que**
l'ARNmisi comprend au moins l'une des séquences SEQIDNO:001, SEQ ID NO : 002 ou SEQ ID NO : 003.

2. Séquence polynucléotidique d'ADN selon la revendication 1, **caractérisée en ce que** la séquence promoteur de spécificité cardiomyocytaire est un promoteur de l'ARN polymérase II.

3. Séquence polynucléotidique d'ADN selon la revendication 1 ou la revendication 2, **caractérisée en ce que** le promoteur de spécificité cardiomyocytaire est au moins l'une des séquences SEQ ID NO : 007, SEQ ID NO : 008 ou SEQ ID NO : 009.

4. Séquence polynucléotidique d'ADN selon l'une quelconque des revendications précédentes, **caractérisée en ce que** le premier élément de séquence comprend en outre des éléments de séquence qui facilitent le traitement du transcrit par l'appareil d'épissage de la cellule.

5. Séquence polynucléotidique d'ADN selon l'une quelconque des revendications précédentes, comprenant au moins l'une des séquences SEQ ID NO : 004 et SEQ ID NO : 005 et au moins l'une des séquences SEQ ID NO : 007, SEQ ID NO : 008 ou SEQ ID NO : 009.

6. Séquence polynucléotidique d'ADN selon l'une quelconque des revendications précédentes, **caractérisée en ce que** la séquence polynucléotidique d'ADN d'origine virale est une séquence d'encapsidation d'adénovirus.

7. Séquence polynucléotidique d'ADN selon l'une quelconque des revendications précédentes, **caractérisée en ce que** la séquence polynucléotidique d'ADN d'origine virale est dérivée d'un virus adéno-associé.

8. Séquence polynucléotidique d'ADN selon la revendication 7, **caractérisé en ce que** la séquence polynucléotidique d'ADN d'origine virale est dérivée d'un virus adéno-associé de type 9.

9. Séquence polynucléotidique d'ADN de SEQ ID NO : 010 ou SEQ ID NO : 011 ou SEQ ID NO : 012.

10. Particule virale contenant une séquence polynucléotidique selon l'une quelconque des revendications précédentes.

11. Cellule eucaryote isolée contenant une séquence polynucléotidique selon l'une quelconque des revendications 1 à 9.

12. Cellule eucaryote isolée selon la revendication 11, de telle manière que la cellule est une cellule de mammifère.

13. Composition pour une utilisation dans le traitement d'une cardiomyopathie comprenant :
a. une séquence polynucléotidique, une particule virale et/ou une cellule de mammifère isolée selon l'une quelconque des revendications 1 à 11 et
b. un excipient pharmaceutiquement acceptable.

14. Composition pour une utilisation dans le traitement d'une cardiomyopathie comprenant :
c. une séquence polynucléotidique ou une particule virale ou une cellule de mammifère isolée selon l'une quelconque des revendications 1 à 11 et
d. une séquence polynucléotidique d'ADN ou une particule virale comprenant une cassette d'expression comprenant une seconde séquence promoteur opérationnelle dans un cardiomyocyte et un second élément de séquence codant pour une SERCA (Ca²⁺-ATPase du réticulum endo/sarcoplasmique) lié de manière opérationnelle à ladite seconde séquence promoteur, et
e. un excipient pharmaceutiquement acceptable.


### SEQUENCE LISTING

<110> Charité Universitätsmedizin Berlin Freie Universität Berlin
<120> Polynucleotide Sequence for the Inhibition of Phospholamban Synthesis
<130> IPA135WO
<160> 13
<170> PatentIn version 3.3
<210> 1
   <211> 64
   <212> DNA
   <213> Homo sapiens
<400> 1
   tgctgtgttg aggcatttca atggttgttt tggccactga ctgacaacca ttgatgcctc 60
   aaca 64
<210> 2
   <211> 64
   <212> DNA
   <213> Homo sapiens
<400> 2
   tgctgataca gatcagcaag agacatgttt tggccactga ctgacatgtc tctctgatct 60
   gtat 64
<210> 3
   <211> 64
   <212> DNA
   <213> Homo sapiens
<400> 3
   tgctgatggt tgaggctctt cttatagttt tggccactga ctgactataa gaagcctcaa 60
   ccat 64
<210> 4
   <211> 28
   <212> DNA
   <213> artificial
<220>
   <223> consensus region flanking 5' of micro-RNA sequence in transcript from which miRNA is released
<400> 4
   ctggaggctt gctgaaggct gtatgctg 28
<210> 5
   <211> 45
   <212> DNA
   <213> artificial
<220>
   <223> consensus region flanking 3' of micro-RNA sequence in transcript from which miRNA is released
<400> 5
   aggacacaag gcctgttact agcactcaca tggaacaaat ggccc 45
<210> 6
   <211> 422
   <212> DNA
   <213> artificial
<220>
   <223> A transcribed sequence comprising a transcription initiation sequence for Pol-II and the transcribed region (the + strand) including splice donor and acceptor sites and the shRNA element
<400> 6
   cagatcgcct ggagaattcg agctcggtac cccggaggta gtgagtcgac cagtggatcc 60
   tggaggcttg ctgaaggctg tatgctgata cagatcagca agagacatgt tttggccact 120
   gactgacatg tctctctgat ctgtatcagg acacaaggcc tgttactagc actcacatgg 180
   aacaaatggc ccagatctgg ccgcatcgag atcgacgcgt gctagaggat cataatcagc 240
   cataccacat ttgtagaggt tttacttgct ttaaaaaacc tcccacacct ccccctgaac 300
   ctgaaacata aaatgaatgc aattgttgtt gttaacttgt ttattgcagc ttataatggt 360
   tacaaataaa gcaatagcat cacaaatttc acaaataaag catttttttc actgcctcta 420
   gc 422
<210> 7
   <211> 2093
   <212> DNA
   <213> homo sapiens
<400> 7
   ggtaccgcgg tggcggccgc ttcgagctcg cccgacattg attattgact agttattaat 60
   agtaatcaat tacggggtca ttagttcata gcccatatat ggagttccgc gttacataac 120
   ttacggtaaa tggcccgcct ggctgaccgc ccaacgaccc ccgcccattg acgtcaataa 180
   tgacgtatgt tcccatagta acgccaatag ggactttcca ttgacgtcaa tgggtggagt 240
   atttacggta aactgcccac ttggcagtac atcaagtgta tcatatgcca agtacgcccc 300
   ctattgacgt caatgacggt aaatggcccg cctggcatta tgcccagtac atgaccttat 360
   gggactttcc tacttggcag tacatctacg tattagtcat cgctattacc atggtgatgc 420
   ggttttggca gtacatcaat gggcgtggat agcggtttga ctcacgggga tttccaagtc 480
   tccaccccat tgacgtcaat gggagtttgt tttggcacca aaatcaacgg gactttccaa 540
   aatgtcgtaa caactccgcc ccatgcggcc gctctagcta gccttgaact cactatgtag 600
   gcaagcatga ccatgaactt ctgatcctcc ttcctcagtg tcctgggata acaggtgtgt 660
   gtcactccct acccttctaa tagcaatatg tggccacatg tttgtgcccc acaggttgag 720
   accatcttga cctgaggaag aaatagctaa cactcacctc ctgaaggttg cctggatctc 780
   gtctttgtct ttccagcact caggagtggg ggggtcagaa gtgcaaagtc agcccctgct 840
   acataatgag ttcaaggctc gcctgggcta catgagacca tgcctcaaaa agaaaaggaa 900
   ttggtatagt gacatactct ggtcctccca gtacttaggg acacagaggc cactccacca 960
   ccatctccag cagctggcct gcctccccga gcctcgttta tttcatatca atgagatggg 1020
   gacccaactg ctaaggtgac cttgcaccca cggggtgact ggagacttga gagtggaggg 1080
   tttatcattt ctccagtcgg tcagcaagtg gtcgccgcca agaaggtttt gagttcaaag 1140
   tagaagatgg gacagggaga gaccagcgag aagaccccac cctggagctg actgtccctg 1200
   tgcggctggg tggggacaca aagcagagaa gcagaggcag agaacaaggg tgggtgacat 1260
   ttgagcaagg atgggggtgt gccagaggct gcccaagatg cataggtgca aaggccctga 1320
   ggttcgagga tgcctggatc cggaatcaaa gctcaggctc ctccctcttc ctcctcctcc 1380
   tctgccccct cctcctcctc tgccccctct tcctcctctg ccccctcttc ttcctcctcc 1440
   tcttcctcct cccctcctca tctacctcct tctcctcctc ctccccctcc tcttcctcct 1500
   ctgccccctc ttcctcctcc tcctcttcct cctcctcttc ctcctcccct cctcatctac 1560
   ctccttctcc tcctcctccc cctcctcttc ctcctctgcc ccctcttcct cctctgcccc 1620
   tcttcctcct cctcctcttc ctcctctgcc ccctcctccc cctcctcttc ctcttcctcc 1680
   tcccctcctc atctacctcc ttctcttcct cctcttcttc ctcctctttc tcctcctcct 1740
   ccctctcctc ttcctcctcc tcttctttct cctcctcctc ttcctccccc tccccttcct 1800
   gggttacttt tccccattag acaatggcag gacccagagc acagagcatc gttcccaggc 1860
   caggccccag ccactgtctc tttaaccttg aaggcatttt tgggtctcac gtgtccaccc 1920
   aggcgggtgt cggactttga acggctctta cttcagaaga acggcatggg gtgggggggc 1980
   ttaggtggcc tctgcctcac ctacaactgc caaaagtggt catggggtta tttttaaccc 2040
   cagggaagag gtatttattg ttccacagca ggggccggcc agcaggctcc ttg 2093
<210> 8
   <211> 1331
   <212> DNA
   <213> homo sapiens
<400> 8
   cggtaccgcg gtggcggccg cttcgagctc gcccgacatt gattattgac tagttattaa 60
   tagtaatcaa ttacggggtc attagttcat agcccatata tggagttccg cgttacataa 120
   cttacggtaa atggcccgcc tggctgaccg cccaacgacc cccgcccatt gacgtcaata 180
   atgacgtatg ttcccatagt aacgccaata gggactttcc attgacgtca atgggtggag 240
   tatttacggt aaactgccca cttggcagta catcaagtgt atcatatgcc aagtacgccc 300
   cctattgacg tcaatgacgg taaatggccc gcctggcatt atgcccagta catgacctta 360
   tgggactttc ctacttggca gtacatctac gtattagtca tcgctattac catggtgatg 420
   cggttttggc agtacatcaa tgggcgtgga tagcggtttg actcacgggg atttccaagt 480
   ctccacccca ttgacgtcaa tgggagtttg ttttggcacc aaaatcaacg ggactttcca 540
   aaatgtcgta acaactccgc cccatgcggc cgctctagcc ggaatcaaag ctcaggctcc 600
   tccctcttcc tcctcctcct ctgccccctc ctcctcctct gccccctctt cctcctctgc 660
   cccctcttct tcctcctcct cttcctcctc ccctcctcat ctacctcctt ctcctcctcc 720
   tccccctcct cttcctcctc tgccccctct tcctcctcct cctcttcctc ctcctcttcc 780
   tcctcccctc ctcatctacc tccttctcct cctcctcccc ctcctcttcc tcctctgccc 840
   cctcttcctc ctctgcccct cttcctcctc ctcctcttcc tcctctgccc cctcctcccc 900
   ctcctcttcc tcttcctcct cccctcctca tctacctcct tctcttcctc ctcttcttcc 960
   tcctctttct cctcctcctc cctctcctct tcctcctcct cttctttctc ctcctcctct 1020
   tcctccccct ccccttcctg ggttactttt ccccattaga caatggcagg acccagagca 1080
   cagagcatcg ttcccaggcc aggccccagc cactgtctct ttaaccttga aggcattttt 1140
   gggtctcacg tgtccaccca ggcgggtgtc ggactttgaa cggctcttac ttcagaagaa 1200
   cggcatgggg tgggggggct taggtggcct ctgcctcacc tacaactgcc aaaagtggtc 1260
   atggggttat ttttaacccc agggaagagg tatttattgt tccacagcag gggccggcca 1320
   gcaggctcct t 1331
<210> 9
   <211> 827
   <212> DNA
   <213> artificial
<220>
   <223> minimum length of heart tissue specific promoter
<400> 9
   gcggccgctt cgagctcgcc cgacattgat tattgactag ttattaatag taatcaatta      60
   cggggtcatt agttcatagc ccatatatgg agttccgcgt tacataactt acggtaaatg     120
   gcccgcctgg ctgaccgccc aacgaccccc gcccattgac gtcaataatg acgtatgttc     180
   ccatagtaac gccaataggg actttccatt gacgtcaatg ggtggagtat ttacggtaaa     240
   ctgcccactt ggcagtacat caagtgtatc atatgccaag tacgccccct attgacgtca     300
   atgacggtaa atggcccgcc tggcattatg cccagtacat gaccttatgg gactttccta     360
   cttggcagta catctacgta ttagtcatcg ctattaccat ggtgatgcgg ttttggcagt     420
   acatcaatgg gcgtggatag cggtttgact cacggggatt tccaagtctc caccccattg     480
   acgtcaatgg gagtttgttt tggcaccaaa atcaacggga ctttccaaaa tgtcgtaaca     540
   actccgcccc atgcggccgc tctaggaccc agagcacaga gcatcgttcc caggccaggc     600
   cccagccact gtctctttaa ccttgaaggc atttttgggt ctcacgtgtc cacccaggcg     660
   ggtgtcggac tttgaacggc tcttacttca gaagaacggc atggggtggg ggggcttagg     720
   tggcctctgc ctcacctaca actgccaaaa gtggtcatgg ggttattttt aaccccaggg     780
   aagaggtatt tattgttcca cagcaggggc cggccagcag gctcctt                   827
<210> 10
   <211> 728
   <212> DNA
   <213> artificial
<220>
   <223> TRE-Tight1 Promotor Konstrukt (Sipo et al. 2006, JMM, 84:215-225) 1-252: TetO7; 253 -314 CMVmin; (t) 306: putative transcriptional start site; 394 - 453: misiPLBh-shRNA; undrlined, italics, 5'
<400> 10
   gagtttactc cctatcagtg atagagaacg tatgtcgagt ttactcccta tcagtgatag     60
   agaacgatgt cgagtttact ccctatcagt gatagagaac gtatgtcgag tttactccct    120
   atcagtgata gagaacgtat gtcgagttta ctccctatca gtgatagaga acgtatgtcg    180
   agtttatccc tatcagtgat agagaacgta tgtcgagttt actccctatc agtgatagag    240
   aacgtatgtc gaggtaggcg tgtacggtgg gaggcctata taagcagagc tcgtttagtg    300
   aaccgtcaga tcgcctggag aattcgagct cggtaccccg gaggtagtga gtcgaccagt    360
   ggatcctgga ggcttgctga aggctgtatg ctgatacaga tcagcaagag acatgttttg    420
   gccactgact gacatgtctc tctgatctgt atcaggacac aaggcctgtt actagcactc    480
   acatggaaca aatggcccag atctggccgc atcgagatcg acgcgtgcta gaggatcata    540
   atcagccata ccacatttgt agaggtttta cttgctttaa aaaacctccc acacctcccc    600
   ctgaacctga aacataaaat gaatgcaatt gttgttgtta acttgtttat tgcagcttat    660
   aatggttaca aataaagcaa tagcatcaca aatttcacaa ataaagcatt tttttcactg    720
   cctctagc                                                             728
<210> 11
   <211> 6535
   <212> DNA
   <213> artificial
<220>
   <223> UFCMV-MLC800-Intron-misiPLBh-pA comprising minimal CMV, micro anti PLB SV40 pA AAV ITRs
<400> 11
   gggggggggg gggggggggg ccactccctc tctgcgcgct cgctcgctca ctgaggccgg 60
   gcgaccaaag gtcgcccgac gcccgggctt tgcccgggcg gcctcagtga gcgagcgagc 120
   gcgcagagag ggagtggcca actccatcac taggggttcc tagatctgaa ttcggtaccg 180
   cggtggcggc cgcttcgagc tcgcccgaca ttgattattg actagttatt aatagtaatc 240
   aattacgggg tcattagttc atagcccata tatggagttc cgcgttacat aacttacggt 300
   aaatggcccg cctggctgac cgcccaacga cccccgccca ttgacgtcaa taatgacgta 360
   tgttcccata gtaacgccaa tagggacttt ccattgacgt caatgggtgg agtatttacg 420
   gtaaactgcc cacttggcag tacatcaagt gtatcatatg ccaagtacgc cccctattga 480
   cgtcaatgac ggtaaatggc ccgcctggca ttatgcccag tacatgacct tatgggactt 540
   tcctacttgg cagtacatct acgtattagt catcgctatt accatggtga tgcggttttg 600
   gcagtacatc aatgggcgtg gatagcggtt tgactcacgg ggatttccaa gtctccaccc 660
   cattgacgtc aatgggagtt tgttttggca ccaaaatcaa cgggactttc caaaatgtcg 720
   taacaactcc gccccatgcg gccgctctag ccggaatcaa agctcaggct cctccctctt 780
   cctcctcctc ctctgccccc tcctcctcct ctgccccctc ttcctcctct gccccctctt 840
   cttcctcctc ctcttcctcc tcccctcctc atctacctcc ttctcctcct cctccccctc 900
   ctcttcctcc tctgccccct cttcctcctc ctcctcttcc tcctcctctt cctcctcccc 960
   tcctcatcta cctccttctc ctcctcctcc ccctcctctt cctcctctgc cccctcttcc 1020
   tcctctgccc ctcttcctcc tcctcctctt cctcctctgc cccctcctcc ccctcctctt 1080
   cctcttcctc ctcccctcct catctacctc cttctcttcc tcctcttctt cctcctcttt 1140
   ctcctcctcc tccctctcct cttcctcctc ctcttctttc tcctcctcct cttcctcccc 1200
   ctccccttcc tgggttactt ttccccatta gacaatggca ggacccagag cacagagcat 1260
   cgttcccagg ccaggcccca gccactgtct ctttaacctt gaaggcattt ttgggtctca 1320
   cgtgtccacc caggcgggtg tcggactttg aacggctctt acttcagaag aacggcatgg 1380
   ggtggggggg cttaggtggc ctctgcctca cctacaactg ccaaaagtgg tcatggggtt 1440
   atttttaacc ccagggaaga ggtatttatt gttccacagc aggggccggc cagcaggctc 1500
   cttgaatttc gaggaactga aaaaccagaa agttaactgg taagtttagt ctttttgtct 1560
   tttatttcag gtcccggatc cggtggtggt gcaaatcaaa gaactgctcc tcagtggatg 1620
   ttgcctttac ttctaggcct gtacggaagt gttacttctg ctctaaaagc tgcggaattg 1680
   tacccgcggc cgcccccaat tcgagctcgc ccggggatcc tctagaatgt caagactgga 1740
   caagagcaaa gtcataaacg gcgctctgga attactcaat ggagtcggta tcgaaggcct 1800
   gacgacaagg aaactcgctc aaaagctggg agttgagcag cctaccctgt actggcacgt 1860
   gaagaacaag cgggccctgc tcgatgccct gccaatcgag atgctggaca ggcatcatac 1920
   ccacttctgc cccctggaag gcgagtcatg gcaagacttt ctgcggaaca acgccaagtc 1980
   attccgctgt gctctcctct cacatcgcga cggggctaaa gtgcatctcg gcacccgccc 2040
   aacagagaaa cagtacgaaa ccctggaaaa tcagctcgcg ttcctgtgtc agcaaggctt 2100
   ctccctggag aacgcactgt acgctctgtc cgccgtgggc cactttacac tgggctgcgt 2160
   attggaggaa caggagcatc aagtagcaaa agaggaaaga gagacaccta ccaccgattc 2220
   tatgccccca cttctgagac aagcaattga gctgttcgac cggcagggag ccgaacctgc 2280
   cttccttttc ggcctggaac taatcatatg tggcctggag aaacagctaa agtgcgaaag 2340
   cggcgggccg gccgacgccc ttgacgattt tgacttagac atgctcccag ccgatgccct 2400
   tgacgacttt gaccttgata tgctgcctgc tgacgctctt gacgattttg accttgacat 2460
   gctccccggg taactaagta aggatccgtc gacaagcttc tgtgccttct agttgccagc 2520
   catctgttgt ttgcccctcc cccgtgcctt ccttgaccct ggaaggtgcc actcccactg 2580
   tcctttccta ataaaatgag gaaattgcat cgcattgtct gagtaggtgt cattctattc 2640
   tggggggtgg ggtggggcag gacagcaagg gggaggattg ggaagacaat agcaggcatg 2700
   ctggggatgc ggtgggctct atggcttctg aggcggaaag aaccagctgg ggtctagtcg 2760
   aggccctttc gtcttcacac gagtttactc cctatcagtg atagagaacg tatgtcgagt 2820
   ttactcccta tcagtgatag agaacgatgt cgagtttact ccctatcagt gatagagaac 2880
   gtatgtcgag tttactccct atcagtgata gagaacgtat gtcgagttta ctccctatca 2940
   gtgatagaga acgtatgtcg agtttatccc tatcagtgat agagaacgta tgtcgagttt 3000
   actccctatc agtgatagag aacgtatgtc gaggtaggcg tgtacggtgg gaggcctata 3060
   taagcagagc tcgtttagtg aaccgtcaga tcgcctggag aattcgagct cggtaccccg 3120
   gaggtagtga gtcgaccagt ggatcctgga ggcttgctga aggctgtatg ctggaaatgt 3180
   actgcgcgtg gagacgtttt ggccactgac tgacgtctcc acgcagtaca tttcaggaca 3240
   caaggcctgt tactagcact cacatggaac aaatggccca gatctggccg catcgagatc 3300
   gacgcgtgct agaggatcat aatcagccat accacatttg tagaggtttt acttgcttta 3360
   aaaaacctcc cacacctccc cctgaacctg aaacataaaa tgaatgcaat tgttgttgtt 3420
   aacttgttta ttgcagctta taatggttac aaataaagca atagcatcac aaatttcaca 3480
   aataaagcat ttttttcact gcctctagca aatcgataac tggggagaga tctaggaacc 3540
   cctagtgatg gagttggcca ctccctctct gcgcgctcgc tcgctcactg aggccgcccg 3600
   ggcaaagccc gggcgtcggg cgacctttgg tcgcccggcc tcagtgagcg agcgagcgcg 3660
   cagagaggga gtggcccccc cccccccccc cccctgcagc ccagctgcat taatgaatcg 3720
   gccaacgcgc ggggagaggc ggtttgcgta ttgggcgctc ttccgcttcc tcgctcactg 3780
   actcgctgcg ctcggtcgtt cggctgcggc gagcggtatc agctcactca aaggcggtaa 3840
   tacggttatc cacagaatca ggggataacg caggaaagaa catgtgagca aaaggccagc 3900
   aaaaggccag gaaccgtaaa aaggccgcgt tgctggcgtt tttccatagg ctccgccccc 3960
   ctgacgagca tcacaaaaat cgacgctcaa gtcagaggtg gcgaaacccg acaggactat 4020
   aaagatacca ggcgtttccc cctggaagct ccctcgtgcg ctctcctgtt ccgaccctgc 4080
   cgcttaccgg atacctgtcc gcctttctcc cttcgggaag cgtggcgctt tctcaatgct 4140
   cacgctgtag gtatctcagt tcggtgtagg tcgttcgctc caagctgggc tgtgtgcacg 4200
   aaccccccgt tcagcccgac cgctgcgcct tatccggtaa ctatcgtctt gagtccaacc 4260
   cggtaagaca cgacttatcg ccactggcag cagccactgg taacaggatt agcagagcga 4320
   ggtatgtagg cggtgctaca gagttcttga agtggtggcc taactacggc tacactagaa 4380
   ggacagtatt tggtatctgc gctctgctga agccagttac cttcggaaaa agagttggta 4440
   gctcttgatc cggcaaacaa accaccgctg gtagcggtgg tttttttgtt tgcaagcagc 4500
   agattacgcg cagaaaaaaa ggatctcaag aagatccttt gatcttttct acggggtctg 4560
   acgctcagtg gaacgaaaac tcacgttaag ggattttggt catgagatta tcaaaaagga 4620
   tcttcaccta gatcctttta aattaaaaat gaagttttaa atcaatctaa agtatatatg 4680
   agtaaacttg gtctgacagt taccaatgct taatcagtga ggcacctatc tcagcgatct 4740
   gtctatttcg ttcatccata gttgcctgac tccccgtcgt gtagataact acgatacggg 4800
   agggcttacc atctggcccc agtgctgcaa tgataccgcg agacccacgc tcaccggctc 4860
   cagatttatc agcaataaac cagccagccg gaagggccga gcgcagaagt ggtcctgcaa 4920
   ctttatccgc ctccatccag tctattaatt gttgccggga agctagagta agtagttcgc 4980
   cagttaatag tttgcgcaac gttgttgcca ttgctacagg catcgtggtg tcacgctcgt 5040
   cgtttggtat ggcttcattc agctccggtt cccaacgatc aaggcgagtt acatgatccc 5100
   ccatgttgtg caaaaaagcg gttagctcct tcggtcctcc gatcgttgtc agaagtaagt 5160
   tggccgcagt gttatcactc atggttatgg cagcactgca taattctctt actgtcatgc 5220
   catccgtaag atgcttttct gtgactggtg agtactcaac caagtcattc tgagaatagt 5280
   gtatgcggcg accgagttgc tcttgcccgg cgtcaatacg ggataatacc gcgccacata 5340
   gcagaacttt aaaagtgctc atcattggaa aacgttcttc ggggcgaaaa ctctcaagga 5400
   tcttaccgct gttgagatcc agttcgatgt aacccactcg tgcacccaac tgatcttcag 5460
   catcttttac tttcaccagc gtttctgggt gagcaaaaac aggaaggcaa aatgccgcaa 5520
   aaaagggaat aagggcgaca cggaaatgtt gaatactcat actcttcctt tttcaatatt 5580
   attgaagcat ttatcagggt tattgtctca tgagcggata catatttgaa tgtatttaga 5640
   aaaataaaca aataggggtt ccgcgcacat ttccccgaaa agtgccacct gacgtctaag 5700
   aaaccattat tatcatgaca ttaacctata aaaataggcg tatcacgagg ccctttcgtc 5760
   tcgcgcgttt cggtgatgac ggtgaaaacc tctgacacat gcagctcccg gagacggtca 5820
   cagcttgtct gtaagcggat gccgggagca gacaagcccg tcagggcgcg tcagcgggtg 5880
   ttggcgggtg tcggggctgg cttaactatg cggcatcaga gcagattgta ctgagagtgc 5940
   accatatgcg gtgtgaaata ccgcacagat gcgtaaggag aaaataccgc atcaggaaat 6000
   tgtaaacgtt aatattttgt taaaattcgc gttaaatttt tgttaaatca gctcattttt 6060
   taaccaatag gccgaaatcg gcaaaatccc ttataaatca aaagaataga ccgagatagg 6120
   gttgagtgtt gttccagttt ggaacaagag tccactatta aagaacgtgg actccaacgt 6180
   caaagggcga aaaaccgtct atcagggcga tggcccacta cgtgaaccat caccctaatc 6240
   aagttttttg gggtcgaggt gccgtaaagc actaaatcgg aaccctaaag ggagcccccg 6300
   atttagagct tgacggggaa agccggcgaa cgtggcgaga aaggaaggga agaaagcgaa 6360
   aggagcgggc gctagggcgc tggcaagtgt agcggtcacg ctgcgcgtaa ccaccacacc 6420
   cgccgcgctt aatgcgccgc tacagggcgc gtcgcgccat tcgccattca ggctacgcaa 6480
   ctgttgggaa gggcgatcgg tgcgggcctc ttcgctatta cgccagctgg ctgca 6535
<210> 12
   <211> 4922
   <212> DNA
   <213> artificial
<220>
   <223> AAV2 MLC 800 heart specific promoter SV40 pA
<400> 12
   cctgcaggca gctgcgcgct cgctcgctca ctgaggccgc ccgggcaaag cccgggcgtc      60
   gggcgacctt tggtcgcccg gcctcagtga gcgagcgagc gcgcagagag ggagtggcca     120
   actccatcac taggggttcc tgcggccgca gaattcggta ccgcggtggc ggccgcttcg     180
   agctcgcccg acattgatta ttgactagtt attaatagta atcaattacg gggtcattag     240
   ttcatagccc atatatggag ttccgcgtta cataacttac ggtaaatggc ccgcctggct     300
   gaccgcccaa cgacccccgc ccattgacgt caataatgac gtatgttccc atagtaacgc     360
   caatagggac tttccattga cgtcaatggg tggagtattt acggtaaact gcccacttgg     420
   cagtacatca agtgtatcat atgccaagta cgccccctat tgacgtcaat gacggtaaat     480
   ggcccgcctg gcattatgcc cagtacatga ccttatggga ctttcctact tggcagtaca     540
   tctacgtatt agtcatcgct attaccatgg tgatgcggtt ttggcagtac atcaatgggc     600
   gtggatagcg gtttgactca cggggatttc caagtctcca ccccattgac gtcaatggga 660
   gtttgttttg gcaccaaaat caacgggact ttccaaaatg tcgtaacaac tccgccccat 720
   gcggccgctc tagccggaat caaagctcag gctcctccct cttcctcctc ctcctctgcc 780
   ccctcctcct cctctgcccc ctcttcctcc tctgccccct cttcttcctc ctcctcttcc 840
   tcctcccctc ctcatctacc tccttctcct cctcctcccc ctcctcttcc tcctctgccc 900
   cctcttcctc ctcctcctct tcctcctcct cttcctcctc ccctcctcat ctacctcctt 960
   ctcctcctcc tccccctcct cttcctcctc tgccccctct tcctcctctg cccctcttcc 1020
   tcctcctcct cttcctcctc tgccccctcc tccccctcct cttcctcttc ctcctcccct 1080
   cctcatctac ctccttctct tcctcctctt cttcctcctc tttctcctcc tcctccctct 1140
   cctcttcctc ctcctcttct ttctcctcct cctcttcctc cccctcccct tcctgggtta 1200
   cttttcccca ttagacaatg gcaggaccca gagcacagag catcgttccc aggccaggcc 1260
   ccagccactg tctctttaac cttgaaggca tttttgggtc tcacgtgtcc acccaggcgg 1320
   gtgtcggact ttgaacggct cttacttcag aagaacggca tggggtgggg gggcttaggt 1380
   ggcctctgcc tcacctacaa ctgccaaaag tggtcatggg gttattttta accccaggga 1440
   agaggtattt attgttccac agcaggggcc ggccagcagg ctccttgaat ttcgaggaac 1500
   tgaaaaacca gaaagttaac tggtaagttt agtctttttg tcttttattt caggtcccgg 1560
   atccggtggt ggtgcaaatc aaagaactgc tcctcagtgg atgttgcctt tacttctagg 1620
   cctgtacgga agtgttactt ctgctctaaa agctgcggaa ttgtacccgc ggccgccccc 1680
   aattcgagct cgcccgggga tcctctagag tcgacctgca gaagcttggt accccggagg 1740
   tagtgagtcg accagtggat cctggaggct tgctgaaggc tgtatgctga tacagatcag 1800
   caagagacat gttttggcca ctgactgaca tgtctctctg atctgtatca ggacacaagg 1860
   cctgttacta gcactcacat ggaacaaatg gcccagatct ggccgcatcg agatcgacgc 1920
   gtgctagagc tagaatcgat aagctagctt ctgtgccttc tagttgccag ccatctgttg 1980
   tttgcccctc ccccgtgcct tccttgaccc tggaaggtgc cactcccact gtcctttcct 2040
   aataaaatga ggaaattgca tcgcattgtc tgagtaggtg tcattctatt ctggggggtg 2100
   gggtggggca ggacagcaag ggggaggatt gggaagacaa tagcaggcat gctggggatg 2160
   cggtgggctc tatggcttct gaggcggaaa gaaccagctg gggtctagag tcccactccc 2220
   tctctgcgcg ctcgctcgct cactgaggcc gggcgaccaa aggtcgcccg acgcccgggc 2280
   tttgcccggg cggcctcagt gagcgagcga gcgcgcagct gcctgcaggg gcgcctgatg 2340
   cggtattttc tccttacgca tctgtgcggt atttcacacc gcatacgtca aagcaaccat 2400
   agtacgcgcc ctgtagcggc gcattaagcg cggcgggtgt ggtggttacg cgcagcgtga 2460
   ccgctacact tgccagcgcc ctagcgcccg ctcctttcgc tttcttccct tcctttctcg 2520
   ccacgttcgc cggctttccc cgtcaagctc taaatcgggg gctcccttta gggttccgat 2580
   ttagtgcttt acggcacctc gaccccaaaa aacttgattt gggtgatggt tcacgtagtg 2640
   ggccatcgcc ctgatagacg gtttttcgcc ctttgacgtt ggagtccacg ttctttaata 2700
   gtggactctt gttccaaact ggaacaacac tcaaccctat ctcgggctat tcttttgatt 2760
   tataagggat tttgccgatt tcggcctatt ggttaaaaaa tgagctgatt taacaaaaat 2820
   ttaacgcgaa ttttaacaaa atattaacgt ttacaatttt atggtgcact ctcagtacaa 2880
   tctgctctga tgccgcatag ttaagccagc cccgacaccc gccaacaccc gctgacgcgc 2940
   cctgacgggc ttgtctgctc ccggcatccg cttacagaca agctgtgacc gtctccggga 3000
   gctgcatgtg tcagaggttt tcaccgtcat caccgaaacg cgcgagacga aagggcctcg 3060
   tgatacgcct atttttatag gttaatgtca tgataataat ggtttcttag acgtcaggtg 3120
   gcacttttcg gggaaatgtg cgcggaaccc ctatttgttt atttttctaa atacattcaa 3180
   atatgtatcc gctcatgaga caataaccct gataaatgct tcaataatat tgaaaaagga 3240
   agagtatgag tattcaacat ttccgtgtcg cccttattcc cttttttgcg gcattttgcc 3300
   ttcctgtttt tgctcaccca gaaacgctgg tgaaagtaaa agatgctgaa gatcagttgg 3360
   gtgcacgagt gggttacatc gaactggatc tcaacagcgg taagatcctt gagagttttc 3420
   gccccgaaga acgttttcca atgatgagca cttttaaagt tctgctatgt ggcgcggtat 3480
   tatcccgtat tgacgccggg caagagcaac tcggtcgccg catacactat tctcagaatg 3540
   acttggttga gtactcacca gtcacagaaa agcatcttac ggatggcatg acagtaagag 3600
   aattatgcag tgctgccata accatgagtg ataacactgc ggccaactta cttctgacaa 3660
   cgatcggagg accgaaggag ctaaccgctt ttttgcacaa catgggggat catgtaactc 3720
   gccttgatcg ttgggaaccg gagctgaatg aagccatacc aaacgacgag cgtgacacca 3780
   cgatgcctgt agcaatggca acaacgttgc gcaaactatt aactggcgaa ctacttactc 3840
   tagcttcccg gcaacaatta atagactgga tggaggcgga taaagttgca ggaccacttc 3900
   tgcgctcggc ccttccggct ggctggttta ttgctgataa atctggagcc ggtgagcgtg 3960
   ggtctcgcgg tatcattgca gcactggggc cagatggtaa gccctcccgt atcgtagtta 4020
   tctacacgac ggggagtcag gcaactatgg atgaacgaaa tagacagatc gctgagatag 4080
   gtgcctcact gattaagcat tggtaactgt cagaccaagt ttactcatat atactttaga 4140
   ttgatttaaa acttcatttt taatttaaaa ggatctaggt gaagatcctt tttgataatc 4200
   tcatgaccaa aatcccttaa cgtgagtttt cgttccactg agcgtcagac cccgtagaaa 4260
   agatcaaagg atcttcttga gatccttttt ttctgcgcgt aatctgctgc ttgcaaacaa 4320
   aaaaaccacc gctaccagcg gtggtttgtt tgccggatca agagctacca actctttttc 4380
   cgaaggtaac tggcttcagc agagcgcaga taccaaatac tgtccttcta gtgtagccgt   4440
   agttaggcca ccacttcaag aactctgtag caccgcctac atacctcgct ctgctaatcc   4500
   tgttaccagt ggctgctgcc agtggcgata agtcgtgtct taccgggttg gactcaagac   4560
   gatagttacc ggataaggcg cagcggtcgg gctgaacggg gggttcgtgc acacagccca   4620
   gcttggagcg aacgacctac accgaactga gatacctaca gcgtgagcta tgagaaagcg   4680
   ccacgcttcc cgaagggaga aaggcggaca ggtatccggt aagcggcagg gtcggaacag   4740
   gagagcgcac gagggagctt ccagggggaa acgcctggta tctttatagt cctgtcgggt   4800
   ttcgccacct ctgacttgag cgtcgatttt tgtgatgctc gtcagggggg cggagcctat   4860
   ggaaaaacgc cagcaacgcg gcctttttac ggttcctggc cttttgctgg ccttttgctc   4920
   ac                                                                  4922
<210> 13
   <211> 281
   <212> DNA
   <213> artificial tight 2 promoter
<220>
   <223> artificial promoter sequence
<400> 13
   gtttactccc tatcagtgat agagaacgta tgtcgagttt actccctatc agtgatagag     60
   aacgatgtcg agtttactcc ctatcagtga tagagaacgt atgtcgagtt tactccctat    120
   cagtgataga gaacgtatgt cgagtttact ccctatcagt gatagagaac gtatgtcgag    180
   tttatcccta tcagtgatag agaacgtatg tcgagtttac tccctatcag tgatagagaa    240
   cgtatgtcga ggtacccggg tcgcctatat aagctggatc c                        281